# EUROPEAN PATENT APPLICATION

(11) **EP 2 506 151 A1**
(43) Date of publication of application: **03.10.2012**
(21) Application number: 09851714.7
(22) Date of filing: 01.12.2009
(51) Int. Cl.: G06F 17/00, G06F 17/30

(54) **SEMANTIC SYNTAX TREE KERNEL-BASED PROCESSING SYSTEM AND METHOD FOR AUTOMATICALLY EXTRACTING SEMANTIC CORRELATIONS BETWEEN SCIENTIFIC AND TECHNOLOGICAL CORE ENTITIES**

(30) Priority: 27.11.2009 KR 20090115936
(71) Applicant: Korea Institute Of Science And Technology Information, Yuseong-gu Daejeon-si 305-333 (KR)
(72) Inventor: CHOI, Sung Pil, Deajeon-si 302-792 (KR); JEONG, Chang Hoo, Daejeon-si 305-801 (KR); CHOI, Yun Soo, Daejeon-si 302-792 (KR); YOON, Hwa Mook, Daejeon-si 305-500 (KR); YOU, Beom Jong, Daejeon-si 305-301 (KR)
(74) Representative: Hodsdon, Stephen James
(86) International application number: PCT/KR2009/007119
(87) International publication number: WO 2011/065617

(57) **Abstract**

The present disclosure relates to a semantic parse tree kernel-based processing system and method of automatically extracting a semantic correlation between scientific and technological core entities. The method includes parsing syntaxes, part-of-speech information, and base chunk information on input sentences; extracting a relation of the syntaxes of the input sentences by performing a parse tree pruning based on the parsed syntaxes, part-of-speech information, and base chunk information; and extracting a similarity of the syntaxes of the input sentences by calculating a syntactic similarity, a lexical semantic similarity, and a semantic parse tree kernel of the syntaxes of the input sentences based on the extracted relations of the syntaxes of the input sentences, thereby more accurately calculating the similarity between two sentences.

## Description

### Technical Field

The present invention relates to a semantic parse tree kernel-based processing system and method, and more particularly, to a semantic parse tree kernel-based processing system and method of automatically extracting a semantic correlation between scientific and technological core entities, which accurately calculates a similarity between two sentences by calculating a semantic parse tree kernel with a syntactic similarity and a lexical semantic similarity.

### Background Art

Protein-Protein Interaction (PPI) includes indirect interaction generated by an aqueous solution, an electrolyte, and the like between hydrated proteins separated from each other by several nanometers, as well as a direct interaction between two adjacent protein molecules.

Information on the PPI may serve an important role in identification and analysis of countless biological functions. Many researchers in a biomedical field have conducted relevant researches through experiments or pursuant to theroretical analysis based on biochemical methods, biophysical methods, etc. until now.

Newly discovered PPI is partially or entirely published in a paper, a patent, a technology report, etc. as a result of a large scaled research, and is mostly expressed in a form of general narrative sentences. Accordingly, an operation for automatically identifying and extracting the PPI information from a text by applying language processing technology, machine learning technology, etc. is very important in two aspects to be detailed below.

First, the operation for automatically identifying and extracting the PPI information is important in an aspect of formalized and systematic collection of recognized PPIs in the past. A database created by collecting and systematically organizing previously discovered PPI may be important basic resources in a new research. Researchers may be aided in determining a starting point and a direction of a research by searching existing resources prior to conduction of their researches. A considerable number of databases have already been built of previously discovered interactions between proteins, genes, or molecules, which are systematically collected and formalized for each field, worldwide.

However, most of the databases are manually established by professionals, and the PPI automatic extraction has been very restrictedly performed. Accordingly, research on the PPI automatic extraction may be considered to be essential in an aspect of more accurate and general collection and utilization of information on the previously discovered PPIs.

Second, the operation for automatically identifying and extracting the PPI information is important in an aspect of the prompt collection of information on newly discovered PPIs. Researchers can immediately submit contents regarding discovered or confirmed PPI to academic conferences or academic journals. However, if the PPIs are mannualy collected by professionals called curators, it takes considerable time to finally register the PPIs to database. Accordingly, PPI automatic extraction technology is very significant in order to maintain the novelty of the PPI database and the prompt expansion of materials.

That is, the research on the PPI automatic extraction has been very actively progressed due to its significance and thus various techniques of the PPI automatic extraction have been introduced. Recently researched PPI extraction methods may be classified into three types, a computational linguistics-based method, a rule-based method, and a machine learning and statistical method, which will be described below.

First, in the computational linquistics-based method, a grammer is constructed by parsing a representative sentence structure capable of expressing the PPI. The structure grammers of components included in the sentence are used as a base grammer of a language parsing system (a part-of-speech tagger, a base chunk recognizer, a syntax parser, etc.) specialized to the PPI extraction.

Second, in the rule-based method, a set of lexical patterns available as a clue of an interaction expression is manually defined and a search for a part corresponding to the lexical pattern in a sentence based on the defined lexical pattern set is performed. One of the rule-based methods is a method of collecting interaction clue word sets, establishing lexical rules based on the collected sets, and applying the lexical rules to the PPI. Accordinly, reliability on the lexical rules discovered within a sentence is automatically calculated and the calculated reliability is utilized as a reliability of the extracted PPI.

Last, in the most recently introduced machine learning and statistical method, a set of qualifications, which are core clues expressing a relation and an interaction, is automatically extracted based on already and manually constructed learning sets by applying a machine learning model based on a supervised learning or a semi-supervised learning and the extracted set of the qualifications is applied to a learning. The machine learning and statistical method shows the highest performance in the extendability and the efficiency, and it has been still actively researched.

Among the aforementioned methods, a kernel-based PPI automatic extraction method has been actively researched. In the kernel-based PPI automatic extraction method, an attempt to extract the PPI was made by defoming a dependent parse tree for candidate sentences to a graph and using a graph kernel in order to overcome a disadvantage of an existing dependent parse tree kernel, but it failed to achieve a better performance than that of the existing PPI extraction method.

Further, an advanced research, in which an experiment was performed on the aforementioned five types of target corpus by constructing a combined kernel employing all of a word qualification kernel, a parse tree kernel, a graph kernel, etc., was conducted. However, in spite of the variety of the applied methods or the application of the broad clue qualifications, the performance of the PPI extraction method using the combined kernel was in a general level.

That is, the parse tree kernel existingly researched for the PPI automatic extraction causes the lowering of the similarity between two actually very similar parse trees resulting from a simple comparision/matching of respective words configuring terminal nodes in the comparision of the two parse trees.

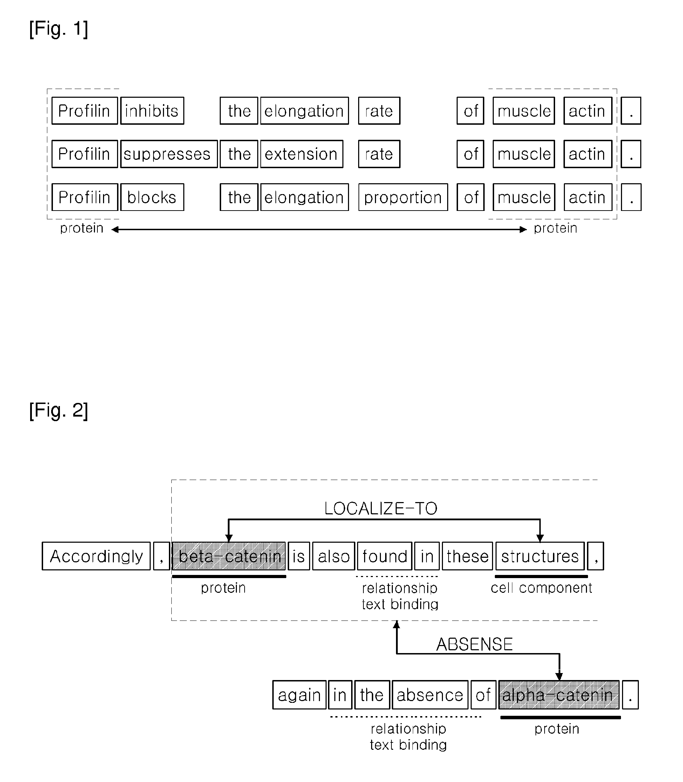

FIG. 1 is an example of a sentence showing a disadvantage of a conventional parse tree kernel. As illustrated in FIG. 1, all of three sentences express the same meaning. Further, in the aspect of the PPI, the three sentences all expresse that "profilin" and "muscle actin" have a "PREVENT" relation.

The syntax structures of the three sentences are the same, so that a kernel value of a pair of any two sentences is expected to be very high (similarity). When an algorithm developed by Moschitti in 2006 is directly applied to the sentences, a lower kernel value than an expected value is obtained due to a simple and external comparison of respective words configuring the terminal nodes.

Accordingly, in order to solve the aforementioned problems and improve a performance of the PPI automatic extraction, a conceptualization is requied for all available words by analyzing meanings of verbs "inhibit", "suppress", and "block" and nouns "elongation", "extension", "rate", and "proportion".

Accordingly, it is necessary to develop a method capable of optimizing a performance of the PPI automatic extraction by developing a semantic parse tree kernel in which the disadvantage of the existing parse tree kernel is improved, applying the developed semantic parse tree kernel to a PPI Classification (PPIC), as well as a PPI Identification (PPII), and applying various learnings and execution options.

### Detailed Description of the Invention

### Technical Problem

Accordingly, the present invention has been made to solve the above-mentioned problems occurring in the prior art, and the present invention relates to a semantic parse tree kernel-based processing system and method for automatic extraction of a semantic correlation between scientific and technological core entities, in which a similarity between two sentences is calculated using a semantic parse tree simultaneously considering a syntactic similarity and a lexical semantic similarity.

### Technical solution

In accordance with an aspect of the present invention, there is provided a semantic parse tree kernel-based processing system for automatically extracting a semantic correlation between scientific technological core entities, the semantic parse tree kernel-based processing system including: a language parser for parsing syntaxes, part-of-speech information, and base chunk information on input sentences; a combined qualification processor for receiving a parse result of the language parser and extracting relations of the syntaxes of the input sentences; and a similarity extracter for extracting a similarity by calculating a syntactic similarity, a lexical semantic similarity, and a semantic parse tree kernel of the syntaxes of the input sentences based on the relations of the syntaxes of the input sentences extracted by the combined qualification processor.

In accordance with another aspect of the present invention, there is provided a semantic parse tree kernel-based processing method of automatically extracting a semantic correlation between scientific and technological core entities, the semantic parse tree kernel-based processing method including the steps of: (a) parsing syntaxes, part-of-speech information, and base chunk information on input sentences by a language parser; (b) extracting a relation of the syntaxes of the input sentences by performing a parse tree pruning based on the syntaxes, the part-of-speech information, and the base chunk information parsed through the language parser by a combined qualification processor; and (c) extracting a similarity of the syntaxes of the input sentences by calculating a syntactic similarity, a lexical semantic similarity, and a semantic parse tree kernel of the syntaxes of the input sentences based on the relations of the syntaxes of the input sentences extracted by the combined qualification processor by a similarity extracter.

### Advantageous effect

According to the semantic parse tree kernel-based processing system and method of automatically extracting a semantic correlation between scientific and technological core entities of the present invention, a syntax, POS information, and base chunk information on input sentences are analyzed, relations between syntaxes of the input sentences are extracted by performing a parse tree pruning based on the analyzed syntaxes, POS information, and base chunk information, and a similarity of the syntaxes of the input sentences is extracted by calculating a syntactical similarity, a lexical semantic similiary, and a semantic parse tree kernel of the input sentences by the extracted relations of the syntaxes of the sentences. Therefore, the present invention may simultaneously utilize the syntactic similairy and the lexical semantic similiary of the two sentences, and more accurately calculate the similiary by preventing the calculation of the improper kernel value due to a homograph and a synonym.

Further, the present invention newly introduces the parse tree kernel decay factor and the word abstraction factor of the semantic parse tree kernel, in addition to the regulization parameter, so that the importance of the decay factor in the application of the parse tree kernel may be identified.

### Brief Description of the Drawings

FIG. 1 is an example of a sentence showing a disadvantage of a conventional parse tree kernel.
FIG. 2 is an example of a sentence including a single PPI of a Biolnfer corpus.
FIG. 3 is an example of a sentence including multiple PPIs of a Biolnfer corpus.
FIG. 4 is a diagram illustrating a symantic parse tree kernel calculation method according to an embodiment of the present invention.
FIG. 5 is a diagram illustrating an example of a path-enclosed tree pruning of a symantic parse tree kernel-based processing system according to an embodiment of the present invention.
FIG. 6 is a block diagram illustrating a symantic parse tree kernel-based processing system according to an embodiment of the present invention.
FIGs. 7 and 8 are flowcharts illustrating a symantic parse tree kernel-based processing method according to an embodiment of the present invention.

Description of reference numerals of main parts in the drawings.
100: symantic parse tree kernel-based processing system
110: language parser
120: combined qualification processor
130: similarity extracter

### Mode for Carrying out the Invention

Terms or words used in the specification and the claims shall not be construed with limitation to a conventional or lexical meaning, and shall be construed with a meaning and a concept coinciding with the technical spirit of the present invention pursuant to a principle that an inventor can properly define concepts of terms for describing his/her invention with the best manner.

Hereinafter, exemplary embodiments of the present invention will be described with reference to the accompanying drawings.

Before the detailed description of a semantic parse tree kernel-based processing method and system for automatic extraction of a semantic correlation between scientific and technological core entities including technical terms and entity names, a Protein-Protein Interaction Extraction (PPIE) will be described below.

The PPIE refers to technology of automatically extracting information on an interaction between multiple proteins appearing witin a text by using a clue word and a syntax structure or other additional qualifications expressed within the text, and includes a PPI Identification (PPII) and a PPI Classification (PPIC).

The PPII is technology for determining whether a sentence or a paragraph including several types of protein names expresses an interaction between the proteins.

In view of the machine learning, a PPII issue may be expressed in a binary classification model, and many researches on the PPII have been progressed.

Further, the PPIC is a work of determining a specific type of interaction through an in-depth parsing of a target sentence or paragraph including the PPI. The number of types of interaction is three or more, so the PPI may be explained with a multiple classification model, and a research on the PPI is continuously ongoing.

Further, the PPII and the PPIC may be expressed in a single multi-classification model by combining the PPII and the PPIC. FIG. 2 is an example of a sentence including a single PPI of a Biolnfer corpus. Referring to FIG. 2, a sentence and an internal structure of the sentence expressing the proteins and the interaction of the proteins can be identified.

The sentence shown in FIG. 2 includes two protein names. As can be seen in FIG. 2, "beta-catenin" that is a sort of cancer-causing protein appearing in the beginning of the sentence is connected with a specific cell component represented in a previous sentence and expressed as "these structures" in the current sentence with a "LOCALIZE-TO" relation. Accordingly, "beta-catenin" and "these structures" do not have the PPI.

However, "alpha-catenin" appearing in the end of the sentence is connected with "beta-catenin" included in the cell component with a relation of "ABSENSE" though there is not the direct relation. Accordingly, "relation text binding" in FIG. 2 is a paragraph clue information essentially explaining the type of PPI.

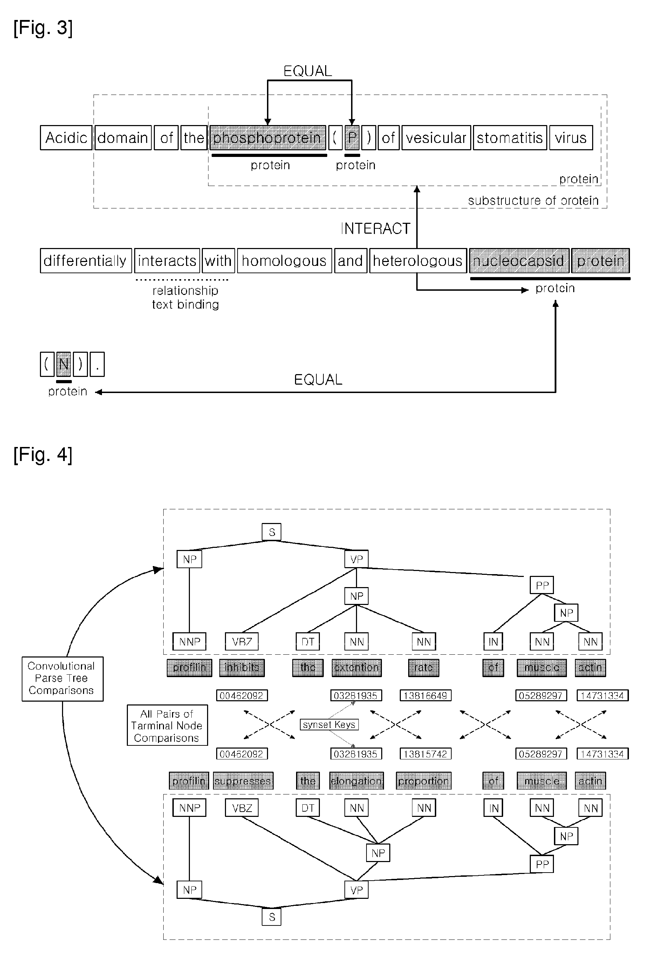

FIG. 3 is an example of a sentence including multiple PPIs of a Biolnfer corpus, and illustrates more complex PPIs than that of FIG. 2. Referring to FIG. 3, "phosphoprotein" is connected with an abbreviation "P" directly adjacent to "phosphoprotein" with an "EQUAL" relation. Further, "phosphoprotein" of "vesicular stomatitis virus" is connected with "nucleocapsid protein, NP" with an "INTERACT" relation.

The nucleocapsid protein which is generally abbreviated as "NP" or "NCP" is abbreviated as "N" in the sentence of FIG. 3 so that "N" is connected to the original term, nucleocapsid protein, with an "EQUAL" relation. Accordingly, the sentence of FIG. 3 has a total of three PPIs.

As can be seen in the aforementioned sentences, it is not easy to extract the PPI from the sentence or paragraph. Especially, as illustrated in FIGs. 2 and 3, a single sentence frequently includes information on two or more interactions. In this situation, it is very difficult to identify different PPIs and classify the type of PPIs based on the same sentence. As mentioned above, if the identification and the classification are manually performed, it is necessary to bring professionals having a high-level language skill, knowledge in a concerned field, and abundant experience.

The present invention according to the embodiment introduces a semantic parse tree kernel expanded from the parse tree kernel previously researched and showing the high performance for the PPI automatic extraction, thereby solving the problem of the existing parse tree kernel in that a kernel value of two semantically similar parse trees is relatively decreased due to a simple and external comparison of respective worlds configuring terminal nodes of the parse tree.

That is, in the semantic parse tree kernel-based processing method and system according to the embodiment of the present invention, a new kernel for simultaneously and effectively calculating a syntactic similarity and a lexical semantic similarity of two parse trees and combining the calculated syntactic similarity and lexical semantic similarity has been conceived.

Namely, a basic concept of the semantic parse tree kernel according to the embodiment of the presnt invention is the extensive application of a semantic similarity to a syntactic similarity between two sentences emphasized in the existing parse tree kernel through the conceptualization of respective words. As a result, a new type of kernel for simultaneously calculating and combining a syntactic similarity and a semantic similarity between two parse trees, is the semantic parse tree kernel.

Although it will be described in more detail, a semantic similarity between two parse trees are calculated by three steps of generating context-based concept information on words included in a separate parse tree, performing a conceptual comparison, rather than a simple superficial comparison, of the words based on the generated context-based concept information, and applying the comparision result to a convolution parse tree kernel.

A basic concept of a convolution parse tree kernel refers to the construction of M-dimensional vector spaces for M number of sub trees by dividing a parse tree into element sub trees and transferring the divided element sub trees to respective axes of the M-dimensional vector spaces. In this case, the separate parse tree is transferred as a specific vector of a vector space. A similarity of a pair of parse tree sets may be measured by calculating inner products of the parse tree sets transferred as the vectors of the vector spaces, and a value of the calculated inner product is an output of the parse tree kernel.

A concept of a context-based word is established by Word Sense Disambiguation (WSD) for words included in terminal nodes, and to this end, a WordNet-based WSD algorithm has been introduced.

Table 1 represents a context-based WSD algorigm using a WordNet. The WSD algorigm will be described in detail with reference to Table 1 below.

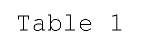

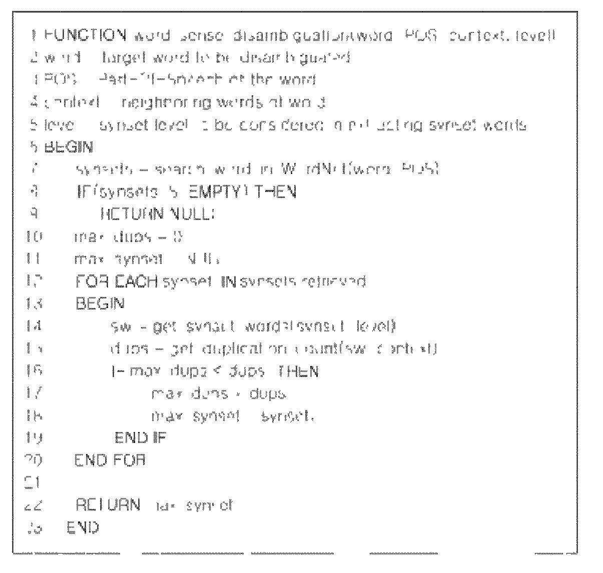

In the WSD algorithm represented in Table 1, an input of the WSD function is a target word, a part of speech of the target word, a neighboring context, and an abstraction level. First, when a target word to be processed is searched for in WordNet, a plurality of synset sets is searched (line 7). This indicates that the target word is expressed in several means in WordNet. A process of selecting a synset most corresponding to context information on the target word among the plurality of synset sets is performed. The process is performed in repetitive sentences from line 12 to line 20. First, component words configuring a specific synset are collected (line 14). A function of "get_synset_words" returns all component words expressing or defining an input synset. The component word includes a synonym set and a gloss. A final factor "level" of the function "get_synset_words" sets a return scope of the component words of the synset.

When the level is "0", only the component words of a current synset are returned, and when the level is "1", component words of a parent synset of the current synset, in addition to the component words of the current synset, are returned. A function "get_duplication_count" calculates a common word between the context information on the target word and the component words of the synset. A synset having the largest number of common words is returned as a final ouput. As a result, a final output value of the WSD algorithm is a specific synset of WordNet.

Table 2 represents input parameters of the semantic parse tree kernel. The semantic parse tree kernel will be described in detail with reference to Table 2 below.

**Table 2**

| Parameter | Variable | Explanation |
|---|---|---|
| Tree kernel decay factor | λ | It is introduced in order to solve kernel value inconsistency generated due to a difference between tree depths of parse trees to be compared. |
| | | It sets a value such that during the comparison of two parse trees, a degree of the contribution of the consistency of kernel values for each node to a kernel value is decreased as a current node becomes close to a terminal node. |
| Tree kernel calculation method setting factor | σ | It sets a parse tree division method for a similarity measurement. |
| | | SubTree, ST |
| | | SubSet Tree, SST |
| Lexical | α | It sets an abstraction level in |
| concept abstraction level setting factor | | generating a concept of a component word of a parse tree. |
| | | 0: Use a synset that is an output by the WSD as it is. |
| | | 1: Use a parent synset of an output synset |
| | | 2: Use a grandparent synset of an output synset |

The semantic parse tree kernel calculating a similarity between two sentences may be expressed as Equation 1. Here, T₁ and T₂ indicate two input parse trees. Three parameters are accompanied for the accurate kernel calculation, the contents of which are represented in Table 2. ${K}_{sem} \left({T}_{1}, {T}_{2}, λ, σ, a\right) = {\sum }_{{n}_{1} ∈ {N}_{{T}_{1}}} {\sum }_{{n}_{2} ∈ {N}_{{T}_{2}}} {Δ}_{sem} \left({n}_{1}, {n}_{2}, λ, σ, a\right)$

As represented in Table 2, the tree kernel decay factor and the kernel calculation method selecting factor are parameters involving the calculation of a syntactic similarity of the semantic parse tree kernel, and the lexical concept abstraction level setting factor is a parameter involving the calculation of a lexical semantic similarity. Finally, function Δₛₑₘ calculates the number of common sub trees of a tree having specific nodes n₁ and n₂ as the highest node, and a detailed algorithm is represented in Table 3.

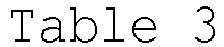

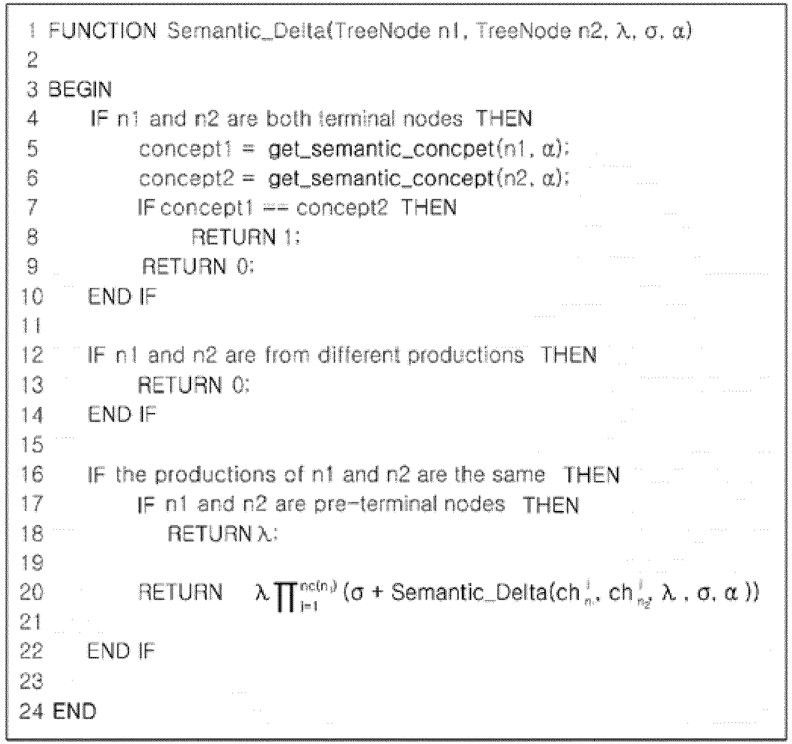

The most pivotal part in the algorithm represented in Table 3 is a part of a lexical concept generation (lines 5 and 6). To this end, a function "get_semantic_concept" returns the synset most proper to a current word by using the WordNet-based WSD algorithm represented in Table 1 and simultaneously performs an abstraction task according to a value of α. A detailed procedure (get_semantic_concept) of the lexical concept generation is as follows.

Extract a set of m left terminal node values and m right terminal node values (context word set) based on a current terminal node value (word)

Extract Part-Of-Speech (POS) information (parent node value) on the current node

Determine a wordnet synset having the highest correlation by calling word_sense_disambiguation (word, context, POS, and level) of Table 1

Move to a parent synset by a number corresponding to α with respect to the determined synset

Return a movement completed synset offset as a lexical concept

The execution of the procedure of the lexical concept generation is limited to a terminal node indicating a word in a parse tree. Regardless of the parse tree decay factors, when two concepts of the words are the same, "1" is returned, and when two concepts of the words are not the same, "0" is returned. The remaining parts of the algorithm represented in Table 3 are publicly disclosed contents, which are the same as those suggested by Moschitti in 2006.

As described above, the semantic parse tree kernel is calculated by simultaneously measuring a syntactic similarity and a lexical semantic similarity between two sentences and combining the measured syntactic similarity and lexical semantic similarity. Accordingly, the kernel function of Equation 1 may be expressed as Equation 2. $\begin{matrix}{K}_{sem} \left({T}_{1}, {T}_{2}, λ, σ, a\right) = {\sum }_{{n}_{1} ∈ {N}_{{T}_{1}}} {\sum }_{{n}_{2} ∈ {N}_{{T}_{2}}} {Δ}_{sem} \left({n}_{1}, {n}_{2}, λ, σ, a\right) \\ = {sim}_{1 ⁢ ex} \left({T}_{1}, {T}_{2}, a\right) + {sim}_{syn} \left({T}_{1}, {T}_{2}, λ, σ\right)\end{matrix}$

Equation 2 represents that the value of the semantic parse tree kernel is calculated by combining the lexical semantic similarity (simlex (T₁, T₂, and α)) and the syntactic similarity (simsyn (T₁, T₂, λ, and σ)) based on Equation 1. The calculation of the lexical semantic similarity is performed only on the terminal node and the calculation of the syntactic similiarty is performed on node other than the terminal node.

A detailed calculation method of the lexical semantic similiarity according to the embodiment of the presneti invention is expressed as Equation 3. $\begin{matrix}{sim}_{1 ⁢ ex} \left({T}_{1}, {T}_{2}, α\right) & = sin \left({W}_{r ⁢ 1}, {W}_{r ⁢ 2}, α\right) & \left(1\right) \\ & \approx Σ \left(Σ sin\left({w}_{1}, {w}_{2}, {c}_{1}, {c}_{2}, α\right)\right) ⁢ {w}_{1} ∈ {W}_{{T}_{1}} , {c}_{1} ⊂ {W}_{{T}_{1}}^{{w}_{1}} ⁢ { w}_{2} ∈ {W}_{{T}_{2}} , {c}_{2} ⊂ {W}_{{T}_{2}}^{{w}_{2}} & \left(2\right) \\ & \approx Σ \left(Σ I \left(concept \left({w}_{1}, {c}_{1}, α\right),concept \left({w}_{2}, {c}_{2}, α\right)\right)\right) ⁢ {w}_{1} ∈ {W}_{{T}_{1}} , {c}_{1} ⊂ {W}_{{T}_{1}}^{{w}_{1}} ⁢ { w}_{2} ∈ {W}_{{T}_{2}} , {c}_{2} ⊂ {W}_{{T}_{2}}^{{w}_{2}} & \left(3\right) \\ & \approx Σ \left(Σ I \left(synset \left({w}_{1}, {c}_{1}, α\right),synset \left({w}_{2}, {c}_{2}, α\right)\right)\right) ⁢ {w}_{1} ∈ {W}_{{T}_{1}} , {c}_{1} ⊂ {W}_{{T}_{1}}^{{w}_{1}} ⁢ { w}_{2} ∈ {W}_{{T}_{2}} , {c}_{2} ⊂ {W}_{{T}_{2}}^{{w}_{2}} & \left(4\right) \\ & \approx Σ \left(Σ I \left(pos\left(synset \left({w}_{1}, {c}_{1}, α\right)\right),pos\left(synset \left({w}_{2}, {c}_{2}, α\right)\right)\right)\right) ⁢ {w}_{1} ∈ {W}_{{T}_{1}} , {c}_{1} ⊂ {W}_{{T}_{1}}^{{w}_{1}} ⁢ { w}_{2} ∈ {W}_{{T}_{2}} , {c}_{2} ⊂ {W}_{{T}_{2}}^{{w}_{2}} & \left(5\right)\end{matrix}$

Here, W_{T} indicates a word set included in the parse tree T, and W_{T}^{ωi} indicates a neighboring context word set among the word set W_{T}.

The lexical semantic similarity between two sentences according to the embodiment of the present invention is defined by the number of common lexical concepts induced through a conceptual cross-comparision of all words included in the two sentences.

The definition is clearly expressed in Equation 3-(1) and 3-(2). Word similarities based on contexts c1 and c2 for all words included in the two sentences are accumulated. Since it is difficult to numerically express and accurately denote the similarity between words, the respective words are conceptualized and the calculation of the similarity between the two words is simplified in Equation 3-(3). In Equation 3-(3), when A is the same as B, "1" is returned, and when A is not the same as B, "0" is returned. As a result, Equation 3-(3) calculates the number of word pairs having the same concept while cross-comparing the two sentences.

To this end, in the embodiment of the present invention, a semantic concept of a specific word is defined as a synset which is most consistent with neighboring context words among the synset sets for the specific word in WordNet. Accordingly, even the same word may represent a different semantic concept depending on a context.

In Equation 3-(4), the synsets of the respective words induced through the WSD algorithm represented in Table 1 are compared based on the definition. Since a specific synset in WordNet may be identified by location information in entire files, the comparision is actually performed based on the location information. pos (S) in Equation 3-(5) returns file offset information on synset S. The abstraction level setting factor α contained in all equations determine abstraction levels of the semantic concepts of the respective words. In the meantime, simsyn (T₁, T2, λ, and σ) indicating the syntactic similarity between the two sentences is the same as that suggested by Moschitti in 2006 and may be expressed as Equation 4. ${sim}_{syn} \left({T}_{1}, {T}_{2}, λ, a\right) = {\sum }_{{n}_{1} ∈ {N}_{{T}_{1}} , {n}_{1} ∉ {L}_{{T}_{1}}} \left({\sum }_{{n}_{1} ∈ {N}_{{T}_{1}} , {n}_{1} ∉ {L}_{{T}_{1}}} Δ\left({n}_{1}, {n}_{2}, λ, σ\right)\right)$

N_{Ti} indicates a set of entire nodes in the parse tree Tᵢ and L_{Ti} indicates a set of entire terminal nodes of Tᵢ. Δ(n₁, n₂, λ, and α) is an algorithm for calculating the number of common sub trees of a tree having specific nodes n₁ and n₂ suggested by Moschitti in 2006 as the highest node.

Based on Equations 3 and 4, the semantic parse tree kernel may be expressed as follows. $\begin{matrix}{K}_{sen} \left({T}_{1}, {T}_{2}, λ, σ, α\right) & = Σ \left(Σ I \left(pos\left(synset \left({w}_{1}, {c}_{1}, α\right)\right),pos\left(synset \left({w}_{2}, {c}_{2}, α\right)\right)\right)\right) ⁢ {w}_{1} ∈ {W}_{{T}_{1}} , {c}_{1} ⊂ {W}_{{T}_{1}}^{{w}_{1}} ⁢ { w}_{2} ∈ {W}_{{T}_{2}} , {c}_{2} ⊂ {W}_{{T}_{2}}^{{w}_{2}} \\ & + Σ \left(Σ Δ\left({n}_{1}, {n}_{2}, λ, σ\right)\right) {n}_{1} ∈ {N}_{{T}_{1}} , {n}_{1} ∉ {L}_{{T}_{1}} {n}_{1} ∈ {N}_{{T}_{1}} , {n}_{1} ∉ {L}_{{T}_{1}}\end{matrix}$

Equation 5 indicates a detailed calculation method of the semantic parse tree kernel. As can be seen from Equation 5, the first section of the right side indicates a lexical semantic similarity, the second section in the right side indicates a syntactic similarity, and a final kernel value is obtained by summing the lexical semantic similarity and the syntactic similarity.

FIG. 4 is a diagram illustrating a symantic parse tree kernel calculation method according to the embodiment of the present invention. Referring to FIG. 4, a lexical semantic similarity for terminal nodes, i.e. component words, is calculated in a center side, and a syntactic similarity is calculated based on two syntax structures in an upper side and a lower side. In this case, the WordNet-based WSD algorithm is applied to the respective words in the center side so that the respective words are conceptualized. Values represented in upper and lower sides of the terminal nodes are identifiers of word concepts, i.e. file offsets in WordNet.

"inhibit" and "suppress" are recongnized to have the same word concept and thus have the same identifier "00462092", but "rate" and "proportion" are recognized to have different concepts, so that different concept identifiers "13816649" and "13815742" are set to "rate" and "proportion", respectively.

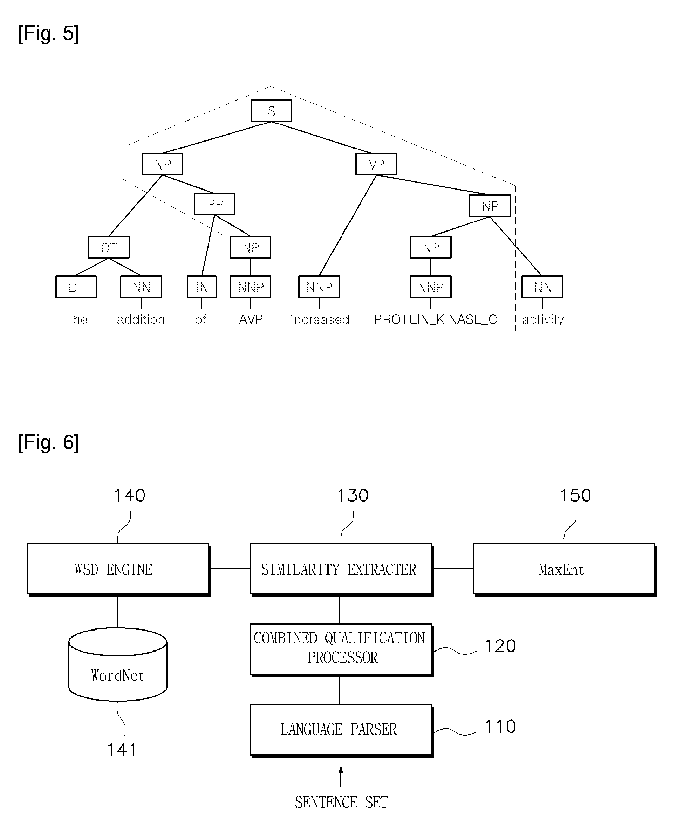

The semantic parse tree kernel-based processing system according to the embodiment of the present invention will be described below with reference to FIGs. 5 and 6.

FIG. 5 is a diagram illustrating an example of a path-enclosed tree pruning of the symantic parse tree kernel-based processing system according to the embodiment of the present invention, and FIG. 6 is a block diagram illustrating the symantic parse tree kernel-based processing system according to the embodiment of the present invention.

The symantic parse tree kernel-based processing system 100 according to the embodiment of the present invention includes a language parser 110, a combined qualification processor 120, and a similarity extracter 130.

The language parser 110 includes a syntax parser, a Conditional Random Field (CRF) chunker, and a CRF POS-tagger. The language parser 110 receives an input of a sentence set, parses syntaxes of input sentences, and tags a POS or recognizes a base chunk.

The combined qualification processor 120 includes a syntax qualification extracter, a word qualification extracter, and an entity qualification extracter.

The syntax qualification extracter receives an input of a syntax parse result of the sentence parsed in the syntax parser, and performs a parse tree pruning by using locations of pre-indicated proteins within the sentence and neighboring syntax information.

The symantic parse tree kernel-based processing system 100 according to the embodiment of the present invention adopts a path-enclosed tree pruning for the parse tree pruning.

The path-enclosed tree pruning is characterized in that the word qualification expressing the interaction between two proteins and the syntax qualification encompassing the word qualification may be simultaneously and concentratively applied.

The word qualification extracter is used for configuration of a general qualification vector by using a word set created within the sentence together with POS information and chunk information generated by the CRF POS tagger and the CRF chunker.

The entity qualification extracter is a module for qualifying intrinsic property information on the proteins and applying the qualified information to the relation extraction when the entity qualification extracter receives the intrinsic property information on the proteins.

The similarity extracter 130 is established based on two types of machine learning models. First, for the Support Vector Machine (SVM)-based relation extraction, libsvm 2.89 is automonously expanded and the parse tree kernel is transplanted to the expanded libsvm 2.89. As a result, a parse tree kernel and and a combined kernel (a kernel combined with a basic kernel and a parse tree kernel) are additionally provided to four basic kernels (linear kernel, multinomial kernel, Radial Basis Function (RBF) kernel, and Sigmoid kernel). The semantic parse tree kernel according to the embodiment of the present invention is accompanied with the WordNet-based WSD system, so is closely related with the WordNet-based WSD system. A WordNEt access API 141 frequently used in the WSD algorithm is configured by modifying/expanding an engine 140 basically provided in the WordNet distribution. Maximum Entropy Modeling Toolkit for Python and C++ (MaxEnt) 150 is used so as to utilize a maximum entropy model.

Further, the respective function element modules are developed in a form of a single package in which the functional element modules are combined and modulated in a source level. Accordingly, the respective function element modules may be applied to various application fields through the setting designation and the addition/change of the module.

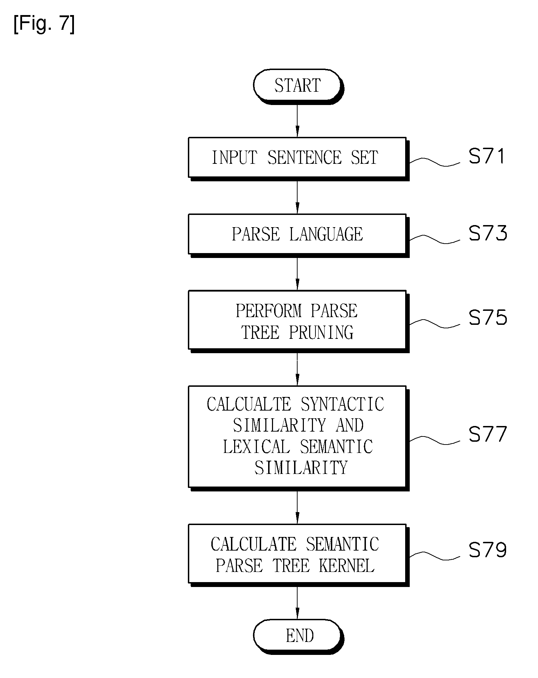

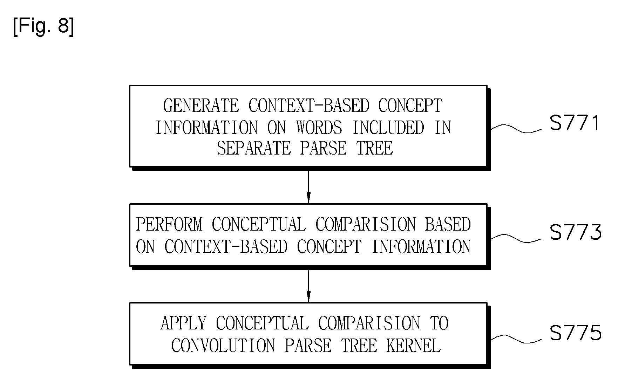

FIGs. 7 and 8 are flowcharts illustrating the symantic parse tree kernel-based processing method according to the embodiment of the present invention. Referring to FIGs. 2 to 8, in step S71, a set of sentences is input to the language parser 110 of the semantic parse tree kernel-based processing system 100 according to the embodiment of the present invention.

In step S73, the language parser 110 parses syntaxes of the input sentences, and parses POS information and chunk information on the input sentences.

In step S75, the combined qualification processor 120 extracts a qualification of the sentence parsed through the language parser 110. Specifically, the syntax qualification extracter receives an input of a syntax parse result of the sentence and performs a parse tree pruning by using locations of pre-indicated proteins within the sentence, and constructs a general qualification vector by using a set of words created within the sentence together with POS information and chunk information parsed through the language parser 110. Further, when intrinsic property information on the proteins is provided, the syntax qualification extracter qualifies the intrinsic property information on the proteins and applies the qualified information to the extraction of the relations of the proteins.

In step S77, the similarity extracter 130 calcualtes a syntactic similarity and a lexical semantic similarity. The syntactic similarity may be calculated by aforementioned Equation 4, and the lexical semantic similarity may be calculated by aforementioned Equation 3.

In step S79, the similarity extracter 130 calcualtes a semantic parse tree kernel based on the syntactic similarity and the lexical semantic similarity calculated in step S77. As expressed in Equation 5, the semantic parse tree kernel may be obtained by summing the syntactic similarity and the lexical semantic similarity.

Specifically, a method of calculating the lexical semantic similarity will be described with reference to FIG. 8.

First, in step S771, the similarity extracter 130 generates context-based concept information on words included in a separate parse tree. In step S773, a conceptual comparision, rather than a simple superficial comparision, is performed on the words based on the generated context-based concept information.

In step S775, the similarity extracter 130 calculates the lexical semantic similarity by applying the conceptual comparision of the words to an existing convolution parse tree kernel.

In order to recognize a performance of the semantic parse tree kernel according to the embodiment of the present invention, a PPI identification test and a PPI classification test were performed by using an existing representative corpus.

Table 4 represents sizes and contents of five PPI corpora used for the performance evaluation of the embodiment of the present invention.

**Table 4**

| Corpus | AIMed | BioInfer | HPRD50 | IEPA | LLL |
|---|---|---|---|---|---|
| Number of sentences | 1,955 | 1,100 | 145 | 486 | 77 |
| Sentence including PPI (Positive instance) | 1,000 | 2,534 | 163 | 335 | 164 |
| Sentence including no PPI (Negative instance) | 4,834 | 7,132 | 270 | 182 | 166 |

Referring to Table 4, the corpora used for the performance evaluation of the present invention is a set of corpora generally called "Five PPI Corpora", and is a collection in which AImed, BioInfer, HPRD50, IEPA, and LLL are converted in a unified XML format. The collection is used as a criterion evaluation collection of the current method of extracting the PPI.

As can be seen in FIGs. 2 and 3, when two or more protein names appear in the specific sentence and the interaction between the proteins is set, a single sentence may include multiple interactions. Further, even though the protein names appear in the sentence, but when the interaction between the proteins is not set, even the sentence including the interaction may be simultaneously set as a sentence including no interaction. Based on this, the PPI extraction may be regulated by a binary classification for a separate instance (a sentence including an interaction/a sentence including no interaction).

Table 5 represents a type of PPI and the number of instances within BioInfer corpora used for the performance evaluation of the embodiment of the present invention.

**Table 5**

| Relations and #Instances | | | | | |
|---|---|---|---|---|---|
| Causal (1,658) | 56 | | | | |
| | Change(1,599) | 205 | | | |
| | | Amount | 39 | | |
| | | Dynamics (190) | 12 | | |
| | | | Full-Stop | 1 | |
| | | | Negative | 48 | |
| | | | Positive | 80 | |
| | | | Start | 7 | |
| | | | Unspecified | 42 | |
| | | Location | 255 | | |
| | | Physical (910) | 8 | | |
| | | | Assembly | 788 | |
| | | | Break-Down | 14 | |
| | | | Modification (100) | 44 | |
| | | | | Addition | 56 |
| | Condition (3) | 3 | | | |
| HUMANMADE (4) | 4 | | | | |
| IS_A (125) | 95 | | | | |
| | Equality (30) | 30 | | | |
| Observation (55) | | 27 | | | |
| | Spatial | 7 | | | |
| | Temporal | 21 | | | |
| PART_OF (318) | Collection:Member | 256 | | | |
| | Object:Component | 62 | | | |
| RELATE (87) | 87 | | | | |

The BioInfer corpora developed in the embodiment of the present invention was used for the PPIC experiment. The BioInfer corpora, which is combined corpora for extraction of information in a biomedical field, is a set in which an individual protein, a protein complex, a protein family or group, a function property, etc., are manually identified for 1,100 sentences extracted from abstracts of research theses in the biomedical field and relations between the proteins are set into 25 types of relations. In the embodiment of the present invention, the experiment was performed on only the individual protein among multiple entities. Table 5 represents a type of interaction and the number of instances for each relation of the BioInfer corpora used in the experiment.

As can be identified in Table 5, respective interactions are hierarchically organized within a BioInfer subset used in the thesis and the number of types of the highest interaction is six. "Assembly" has the largest number of 788 instances based on a terminal interaction, and "Full-Stop" merely has one instance. Taken as a whole, the imbalanced concentration of the instance is serious and the instances are not regularly distributed. Accordingly, it is highly possible that a problem of data sparseness is generated in the automatic classification.

Further, the terminal nodes configuring relation ontology of the BioInfer corpus includes a relation predicate. The relation predicate indicates a verb or a verb phrase in various forms expressing a specific relation. Accordingly, if the relation ontology is taken as a single tree form, the tree may be classified into a node representing a relation type class and a node representing a relation predicate represented in Table 5.

**Table 6**

| Level | 1 | 2 | 3 | 4 | 5 | 6 | #Total |
|---|---|---|---|---|---|---|---|
| #Relation Type Classes | 4 | 8 | 6 | 8 | 2 | 0 | 28 |
| #Relation Predicates | 4 | 6 | 20 | 7 | 26 | 5 | 68 |
| #Total | 8 | 14 | 26 | 15 | 28 | 5 | 96 |

Table 6 represents the number of nodes for each level in the BioInfer relation ontology. As represented in Table 6, the BioInfer relation ontology includes 96 nodes in a total of 6 levels, the number of relation type classes is 28 and the number of relation predicates is 68. The experiment was performed using a total of four types of corpora including corpora including a total of 25 relation type classes, corpora including relation sets in the highest level, corpora including relation sets up to level 2, and corpora including relation sets up to level 3, based on sets of relations represented in Table 5. Although it is not represented in Table 5, the experiment used the relation sets including the relation predicate.

Table 7 represents a type and the number of relation sets used in the experiment.

**Table 7**

| Relation set | Explanation | Number of relations |
|---|---|---|
| RCP_L1 | Relation set in the highest level | 6 |
| RCP_L2 | Relation set up to the second level | 12 |
| RCP_L3 | Relation set up to the third level | 22 |
| RCO | Relation set including only relation classes | 25 |

The embodiment of the present invention uses four types of relation sets having different granularities in order to measure a performance change according to a size of the relation types. The result of the performance measurement is as follows.

The experiment performed in the embodiment of the present invention involves a total of three parameters, and the performance may be differentiated according to a method of setting the parameter. Table 8 represents a type and the number of settings of a target system for the experiment.

**Table 8**

| Parameter | Details | Rrange | | Number of setting |
|---|---|---|---|---|
| λ | Parse tree kernel decay factor | 0.1~1.0(unit: 0.1) | | 10 |
| C | SVM regularization parameter | 1.0~7.0(unit: 1.0) | | 7 |
| α | Abstraction level setting factor for word concept in semantic parse 2 tree kernel | 0 | Use a node concept as it is | |
| | | 1 | Use a parent of a current node concept | |
| | | 2 | Use a grandparent of a current node concept | 4 |
| | | N | Existing parse tree kernel | |
| Total number of systems | | | | 280 |

10 parse tree kernel decay factors are set from a minimum 0.1 to a maximum 1.0 by a unit of 0.1. The number of SVM regularization factors is limited to 7 from 1.0 to 7.0. Lastly, the number of the types of word concept abstraction level setting factor described in Table 2 is set as 4. Accordingly, when all three parameters are applied, a total of 280 settings are induced. In the embodiment of the present invention, the performance is measured by performing 10-fold cross invalidation on each setting set. A performance measurement reference used in the semantic parse tree kernel-based processing system and method according to the embodiment of the present invnetion is a macro-averaged F-score and a micro-averaged F-score. In a macro-average based method, an F-score is calculated by adding precisions and recals of respective m classes and dividing the calculated F-scores by m. In a micro-average based method, an F-score is calculated based on the accumulation of correctly classified data and incorrectly classified data based on entire validation data. The macro-average based method has an advantage in that the classification performance of a learning model for all classes may be generally examined, but has a disadvantage of a relatively low performance measurement result when the learning sets for each class are not evenly distributed. The micro-average based method has a disadvantage in that when the classification performance for a specific class of a leaning model is relatively low, the specific class is not properly reflected. When the distribution of the learning sets for each class is different or a prediction performance of a learning model for a specific class is low, a difference in values according to the two evaluation methods is sometimes considerable.

**Table 9**

| Collection | Tree Kernels | Abstraction Level | DF(λ) | Regularization Factor(C | mi-F1 | Precision | Recall | ma-F1 |
|---|---|---|---|---|---|---|---|---|
| AIMed | SPTK | 1 | 0.5 | 7.0 | 89.33 | 84.86 | 77.45 | 80.99 |
| BioInfer | SPTK | 0 | 0.5 | 5.0 | 89.00 | 87.22 | 84.81 | 86.00 |
| IEPA | PTK | | 0.4 | 7.0 | 79.12 | 78.51 | 78.30 | 78.41 |
| HPRD50 | SPTK/PT | 0/1/2 | 0.7 | 6.0 | 85.22 | 84.74 | 83.41 | 84.07 |
| | K | | | | | | | |
| LLL | SPTK | 1 | 0.4 | 4.0 | 88.48 | 88.64 | 88.47 | 88.55 |

Table 9 represents setting values and detailed performance information representing the highest performance for each corpus. Here, ma-F1 is an abbreviation of macro-averaged F1, mi-F1 is an abbreviation of micro-averaged F1, SPTK is an abbreviation of and indicates a Semantic Parse Tree Kernel, and PTK is an abbreviation of and indicates a Parse Tree Kernel.

Referring to Table 9, settings and detailed performance values showing the highest performance among the total of 280 systems are represented for each of 5 corpora. In an experiment for 5 target corpora, the SPTK showed the highest performance in the remaining corpora except for IEPA. HPRD50 showed the same performance in the PTK and the SPTK. The word concept abstraction level showed the highest performace in "0" or "1" and showed the performance degradation in "2" compared to a general PTK. The remaining corpora except for "IPEA" showed 80.0 or higher based on the macro-averaged F-score.

**Table 10**

| Collections | PTK | SPTK | | | SPTK(total) | Coverage rate |
|---|---|---|---|---|---|---|
| | | α=0 | α=1 | α=2 | | |
| AIMed | 7 | 4 | 5 | 4 | 13 | 65% |
| BioInfer | 3 | 7 | 5 | 5 | 17 | 85% |
| IEPA | 12 | 4 | 3 | 1 | 8 | 40% |
| HPRD50 | 6 | 4 | 4 | 6 | 14 | 70% |
| LLL | 4 | 5 | 5 | 6 | 16 | 80% |

Table 10 represents a setting distribution in 20 higher systems based on the ma-F1. Referring to Table 10, there were a large number of occurances of the SPTK in the remaining corpora except for "IEPA". In the BioInfer corpora, 17 systems out of 20 systems were the SPTK. Accordingly, it can be seen that the semantic parse tree kernel-based method generally maintains the high performance.

**Table 11**

| Target system | AIMed | BioInfer | HPRD50 | IEPA | LLL | Average |
|---|---|---|---|---|---|---|
| Airola et al. (2008) | 56.4 | 61.3 | 63.4 | 75.1 | 76.8 | 66.60 |
| Miwa et al. (2009) | 60.8 | 68.1 | 70.9 | 71.7 | 80.1 | 85.52 |
| Our system(PTK, λ = 0.4) | 75.4 | 81.2 | 77.9 | 175.1 | 85.5 | 79.02 |
| Our system (SPTK, α = 0, λ = 0.4) | 75.5 | 81.4 | 77.9 | 75.6 | 85.2 | 79.12 |
| Our system(SPTK, α = 1, λ = 0.4) | 75.2 | 81.3 | 77.9 | 75.1 | 85.2 | 78.94 |
| Our system (SPTK, α = 2, λ = 0.4) | 74.8 | 81.2 | 77.9 | 75.1 | 85.5 | 78.90 |

Finally, Table 11 represents results of comparision and analysis of the performance of the embodiment of the present invention and the previous research results. Referring to Table 11, for the comparision in the same condition, the same regularization factors as the existing research were applied and all tree kernel decay factors were fixed to 0.4.

As can be seen in Table 11, the systems showing the highest performance in the embodiment of the present invention averagely showed the higher performance than the systems of the previous researches. The performance in the embodiment of the present invention may be considered to be improved by three reasons. The first reason is the application of the parse tree pruning. As described above, when multiple protein names appear within the same sentence, the interaction expression qualitfications for respective pairs of different protein names in the sentence may be differenciated. For example, in a sentence "PROT_A inhibits PROT_B that increases PROT_C's activity", the interaction between "PROT_A" and "PROT_B" is expressed with a verb "inhibits", but the interaction between "PROT_B" and "PROT_C" is expressed with a verb "increases". If the parse tree pruning is not performed, the syntactic qualification is equally applied to the two pairs of proteins, even though there is no direct relation between "PROT_A" and "PROT_C", it may be determined that the two proteins have the interaction.

Accordingly, the parse tree pruning is essential in the application of the parse tree kernel, and it may be estimated that the reason of the poor performance improvement even though the combination and the application of the various kernels including the parse tree kernel in the previous researches is the non-application of the parse tree pruning. The second reason is the performance optimization by the decay factor of the parse tree kernel. The decay factor basically serves as a smoothing value in a parse tree kernel function. That is, rather than accurately calculating the similarity of the parse tree up to the terminal nodes, the decay factor serves to control the kernel contribution of a sub tree having serious deformation and many syntactic errors according to the deformation in a predetermined level, so that the diversity of the learning model results in improvement.

Although the system, which uses the semantic parse tree kernel and does not perform the abstraction of the word concept, showed the highest performance, the performance improvement was not noticeable compared to other systems. This shows that the PPII that is the binary classification does not have a high difficulty to the extent of showing a noticeable performace through the application of the semantic parse tree kernel. A peculiar point is that as the abstraction level for the word concept increases, the performance is rather decreased. This shows a relation with a phenomenon in which as the abstraction level increases, the levels of semantic difficulties of respective words are decreased.

**Table 12**

| RelationSet | Tree Kernels | Abstra ction Level | DF(λ) | Regularization Factor(C) | mi-F1 | Precision | Recall | ma-F1 |
|---|---|---|---|---|---|---|---|---|
| RCP_L1 | SPTK | 2 | 0.3 | 5 | 91.63 | 75.05 | 63.03 | 68.51 |
| RCP_L2 | SPTK | 0 | 0.2 | 7 | 90.52 | 76.65 | 60.27 | 67.48 |
| RCP_L3 | SPTK | 1 | 0.1 | 4 | 78.06 | 71.86 | 52.65 | 60.77 |
| RCO | SPTK | 0 | 0.4 | 5 | 78.02 | 75.46 | 57.74 | 65.42 |

Table 12 represents setting values showing the highest performance and performance detailed information for each relation set. As can be seen in Table 12, the performance of the semantic parse tree kernel is highest in all relation sets. The performance of the entire 25 relation sets except for the relation predicate is 65.4 based on the macro-averaged F-score. As can be seen in Table 12, a difference between the micro-averaged F-score and the macro-averaged F-score is definite. Especially, the micro-averaged F-score in RCP_L1 including 6 highest classification sets shows a very high value of 91.63, but the macro-averaged F-score is merely 68.51. This is caused by the instance imbalanced concentraction for each component relation as pointed out in Table 5. The distribution of the systems within 30^{th} place in a higher order based on the macro-averaged F-score is analyzed in order to review the performance improvement degree of the semantic parse tree kernel in depth as suggested in Table 1 and the analysis result is represented in Table 13.

**Table 13**

| Collections | PTK | SPTK | | | PTK SPTK(total) Coverage | rate |
|---|---|---|---|---|---|---|
| | | α=0 | α=1 | α=2 | | |
| RCP_L1 | 8 | 9 | 7 | 6 | 22 | 73.3% |
| RCP_L2 | 9 | 8 | 7 | 6 | 21 | 70.0% |
| RCP_L3 | 8 | 9 | 7 | 6 | 22 | 73.3 |
| RCO | 5 | 9 | 9 | 7 | 25 | 83.3% |

Table 13 represents the distribution of the settings in the higher 30 systems based on the ma-F1. Referring to FIG. 13, the semantic parse tree kernel shows the high share for all relation sets. Especially, for the RCO set, 25 out of 30 higher systems share the semantic parse tree and thus the semantic parse tree makes up a very high share in the setting distribution of the higher systems. Such a phenomenon is very encouraging in an aspect that the RCO is a single complete classificaiotn system, whereas the remaining relation sets are somewhat abnormal classification systems in which the relation type class and the relation predicate coexist.

Finally, in order to more accurately compare the performance between the general PTK and the SPTK, the performance of the two kernels were compared based on the same setting showing the highest performance of the PTK, and the result is represented in Table 14.

**Table 14**

| Relation set (setting) | Type of tree kernel | mi-F1 | Precision | Recall | ma-F1 |
|---|---|---|---|---|---|
| RCP_L1 (λ=0.3, C=7.0) | PTK | 91.94 | 75.68 | 62.23 | 68.30 |
| | SPTK (α=0) | 91.90 | 75.63 | 62.37 | 68.36 |
| | SPTK (α=1) | 91.72 | 75.55 | 62.29 | 68.28 |
| | SPTK (α=2) | 91.68 | 75.02 | 61.72 | 67.72 |
| RCP_L2 (λ=0.2, C=5.0) | PTK | 89.99 | 76.47 | 58.91 | 66.55 |
| | SPTK (α=0) | 89.90 | 76.43 | 58.80 | 66.47 |
| | SPTK (α=1) | 89.90 | 76.61 | 58.88 | 66.58 |
| | SPTK (α=2) | 89.81 | 76.66 | 58.03 | 66.06 |
| RCP_L3 (λ=0.1, C=4.0) | PTK | 78.02 | 71.84 | 51.81 | 60.20 |
| | SPTK (α=0) | 78.10 | 71.65 | 52.73 | 60.75 |
| | SPTK (α=1) | 78.06 | 71.86 | 52.65 | 60.77 |
| | SPTK (α=2) | 77.53 | 71.29 | 51.54 | 59.83 |
| RCO (λ=0.4, C=5.0) | PTK | 77.88 | 74.90 | 57.05 | 64.77 |
| | SPTK (α=0) | 78.02 | 75.46 | 57.74 | 65.42 |
| | SPTK (α=1) | 77.84 | 75.50 | 57.51 | 65.29 |
| | SPTK (α=2) | 77.44 | 74.96 | 57.09 | 64.82 |

As represented in Table 14, the difference of the performance is not remarkable, but it can be noted that the SPTK shows the better performace in all relation sets.

As a result, the semantic parse tree kernel was invented by expanding the calculation of the kernel value while focusing on only the syntactic similarity between the two sentences in the existing parse tree kernel and simultaneously applying the lexical semantic similarity. To this end, the context and WordNet-based WSD system and the abstraction of the WordNet synset induced as an output of the context and WordNet-based WSD system are simultaneously applied to the existing PPII and PPIC. The context-based WSD in the kernel function serves as follows. First, a syntactic similarity and a lexical semantic similarity between two target sentences may be simultaneously utilized. Second, the calculation of an improper kernel value due to a homograph and a synonym may be partically prevented, so that a more accurate similarity calculation may be achieved. Finally, since a word concept is set using WordNet, an entire system optimization in a specific region may be supported by additionally introducing a word concept abstraction level factor using a hierarchy structure of WordNet.

In relation to the final role of the context-based WSD, in order to achieve the in-depth optimization of the PPII and the PPIC, the experiment of the embodiment of the present invention newly introduced a decay factor of the parse tree kernel and a word abstraction factor of the semantic parse tree kernel. Through the experiment, it was experimentally shown that the function of the decay factor is important in the application of the parse tree kernel and the semantic parse tree kernel may aid in the performance improvement of the existing system. Especially, it could be identified that the semantic parse tree kernel is more effective to the PPIC, compared to the PPII having a relatively low level of difficulty. Further, it also could be identified that the word abstraction factor of the semantic parse tree kernel was the important factor for the performance improvement together with the decay factor.

Although a preferred embodiment of the present invention has been described for illustrative purposes, those skilled in the art will appreciate that various modifications and implementations are possible, without departing from the scope and spirit of the invention as disclosed in the accompanying claims.

## Claims

1. A semantic parse tree kernel-based processing system for automatically extracting a semantic correlation between scientific technological core entities, the semantic parse tree kernel-based processing system comprising:
a language parser for parsing syntaxes, part-of-speech information, and base chunk information on input sentences;
a combined qualification processor for receiving a parse result of the language parser and extracting relations of the syntaxes of the input sentences; and
a similarity extracter for extracting a similarity by calculating a syntactic similarity, a lexical semantic similarity, and a semantic parse tree kernel of the syntaxes of the input sentences based on the relations of the syntaxes of the input sentences extracted by the combined qualification processor.

2. The semantic parse tree kernel-based processing system as claimed in claim 1, wherein the combined qualification processor extracts the relation of the syntaxes of the input sentences by a parse tree pruning of analyzing existence or nonexistence of an interaction between two proteins with a syntax qualification and a word qualification expressing the interaction between the two proteins.

3. The semantic parse tree kernel-based processing system as claimed in claim 2, wherein the language parser comprises:
a syntax parser for parsing the syntaxes of the input sentences;
a Conditional Random Field (CRF) Part-Of-Speech (POS)-tagger for parsing part-of-speech information on the syntaxes of the input sentences; and
a CRF chunker for recognizing base chunks of the syntaxes of the input sentences.

4. The semantic parse tree kernel-based processing system as claimed in claim 3, wherein the combined qualification processor comprises:
a syntax qualification extracter for receiving a syntax parse result for the input sentences from the language parser and performing the parse tree pruning by using locations of the proteins within the input sentences and neighboring syntax information;
a word qualification extracter for configuring a general qualification vector by using a word set created within the sentences together with the part-of-speech information and the base chunk information on the input sentences received through the language parser; and
an entity qualification extracter for qualifying intrinsic property information on the proteins and applying the qualified intrinsic property information to correlation extraction when the intrinsic property information is provided.

5. The semantic parse tree kernel-based processing system as claimed in one of claims 1 to 4, wherein the lexical semantic similarity is a number of common word concepts induced through a conceptual cross comparision of all words included in two sentences.

6. A semantic parse tree kernel-based processing method of automatically extracting a semantic correlation between scientific and technological core entities, the semantic parse tree kernel-based processing method comprising the steps of:
(a) parsing syntaxes, part-of-speech information, and base chunk information on input sentences by a language parser;
(b) extracting a relation of the syntaxes of the input sentences by performing a parse tree pruning based on the syntaxes, the part-of-speech information, and the base chunk information parsed through the language parser by a combined qualification processor; and
(c) extracting a similarity of the syntaxes of the input sentences by calculating a syntactic similarity, a lexical semantic similarity, and a semantic parse tree kernel of the syntaxes of the input sentences based on the relations of the syntaxes of the input sentences extracted by the combined qualification processor by a similarity extracter.

7. The semantic parse tree kernel-based processing method as claimed in claim 6, wherein the similarity extracter calculates the lexical semantic similarity by generating context-based concept information on words configuring a separate parse tree of the syntaxes of the input sentences, performing a conceptual comparison on the words based on the generated context-based concept information, and applying the conceptual comparision to a convolution parse tree kernel.

8. The semantic parse tree kernel-based processing method as claimed in claim 7, wherein the syntactic similarity is calculated by an equation ${sim}_{syn} \left({T}_{1}, {T}_{2}, λ, a\right) = {\sum }_{{n}_{1} ∈ {N}_{{T}_{1}} , {n}_{1} ∉ {L}_{{T}_{1}}} \left({\sum }_{{n}_{1} ∈ {N}_{{T}_{1}} , {n}_{1} ∉ {L}_{{T}_{1}}} Δ\left({n}_{1}, {n}_{2}, λ, σ\right)\right) ,$
wherein Tᵢ indicates a parse tree, λ indicates a tree kernel decay factor, σ indicates a tree kernel calculation method designating factor, n₁ and n₂ indicate specific nodes, N_{Ti} indicates a set of entire nodes of the parse tree Tᵢ, and L_{Ti} indicates a set of all terminal nodes of the parse tree Tᵢ.

9. The semantic parse tree kernel-based processing method as claimed in claim 8, wherein the lexical semantic similarity is calculated by equations ${sim}_{1 ⁢ ex} \left({T}_{1}, {T}_{2}, α\right) = sin \left({W}_{{r}_{1}}, {W}_{{r}_{2}}, α\right)$ $\approx {\sum }_{{w}_{1} ∈ {W}_{{T}_{1}} , {c}_{1} ⊂ {W}_{{T}_{1}}^{{w}_{1}}} \left({\sum }_{{w}_{1} ∈ {W}_{{T}_{2}} , {c}_{2} ⊂ {W}_{{T}_{2}}^{{w}_{2}}}sim\left({w}_{1}, {w}_{2}, {c}_{1}, {c}_{2}, a\right)\right)$ $\begin{matrix}\approx Σ \left(Σ I \left(concept \left({w}_{1}, {c}_{1}, α\right),concept \left({w}_{2}, {c}_{2}, α\right)\right)\right) ⁢ {w}_{1} ∈ {W}_{{T}_{1}} , {c}_{1} ⊂ {W}_{{T}_{1}}^{{w}_{1}} ⁢ { w}_{2} ∈ {W}_{{T}_{2}} , {c}_{2} ⊂ {W}_{{T}_{2}}^{{w}_{2}}\end{matrix}$ $\begin{matrix}\approx Σ \left(Σ I \left(synset \left({w}_{1}, {c}_{1}, α\right),synset \left({w}_{2}, {c}_{2}, α\right)\right)\right) ⁢ {w}_{1} ∈ {W}_{{T}_{1}} , {c}_{1} ⊂ {W}_{{T}_{1}}^{{w}_{1}} ⁢ { w}_{2} ∈ {W}_{{T}_{2}} , {c}_{2} ⊂ {W}_{{T}_{2}}^{{w}_{2}}\end{matrix}$ $\begin{matrix}\approx Σ \left(Σ I \left(pos\left(synset \left({w}_{1}, {c}_{1}, α\right)\right),pos\left(synset \left({w}_{2}, {c}_{2}, α\right)\right)\right)\right) ⁢ {w}_{1} ∈ {W}_{{T}_{1}} , {c}_{1} ⊂ {W}_{{T}_{1}}^{{w}_{1}} ⁢ { w}_{2} ∈ {W}_{{T}_{2}} , {c}_{2} ⊂ {W}_{{T}_{2}}^{{w}_{2}}\end{matrix}$
wherein Tᵢ indicates the parse tree, α indicates a word concept abstraction level setting factor, W_{T} indicates a set of words included in the parse tree T, and W_{T}^{ωi} indicates a set of neighboring syntax words of ωᵢ among the set of words Wₜ.

10. The semantic parse tree kernel-based processing method as claimed in claim 9, wherein in the step (c), the similarity extracter calculates the semantic parse tree kernel with a sum of the syntactic similarity and the lexical semantic similarity.
